# EUROPEAN PATENT SPECIFICATION

(11) **EP 2 801 321 B1**
(45) Date of publication and mention of the grant of the patent: **24.08.2016**
(21) Application number: 14177459.6
(22) Date of filing: 16.11.2012
(51) Int. Cl.: A61B 6/14, G01T 1/24, H01L 27/146, H05K 5/02

(54) **Intraoral radiographic imaging sensors with minimized mesial imaging dead space**
Intraorale Röntgenabbildungssensoren mit minimiertem mesialem Abbildungs-Eigenraum
Capteurs d'imagerie de radiographie intra-orale avec un minimum d'espace mort d'imagerie mésiale

(30) Priority: 18.11.2011 US 201161561476 P; 09.11.2012 US 201213673763
(43) Date of publication of application: 12.11.2014
(62) Divisional of application: 12193006.9
(73) Proprietor: Cyber Medical Imaging, Inc., Los Angeles, CA 90064 (US)
(72) Inventor: Yoon, Douglas C., Beverly Hills, CA California 90210 (US); Chen, Adam, Pacific Palisades, CA California 90272 (US)
(74) Representative: Viering, Jentschura & Partner mbB Patent- und Rechtsanwälte

(56) References cited:
- EP-A1- 1 623 673
- WO-A1-96/32064
- US-A- 5 510 623
- US-A- 6 069 935

## Description

### Field of the Invention

The present invention is in the field of intraoral radiographic imaging sensors and their methods of use and, more particularly, to increasing patient comfort during such use.

### Background of the Invention

Radiographs are fundamental to most dental diagnostic procedures. However, a common complaint and problem during radiographic exams is patient discomfort during the placement of radiographic sensors within the mouth. The majority of these complaints involve the placement of the radiographic sensor in the posterior region of the maxillary and mandibular arches of the patient. This problem is primarily due to the limited space available for proper placement of the sensors within these regions. This has been a problem since the inception of dental radiography using standard x-ray film technology.

Recently, solid-state x-ray sensors have been developed that replace film. The patient discomfort problem for these sensors is even greater because these devices are rigid by nature and cannot be bent like film to conform to the patient's anatomy.

As noted in U.S. Patent No. 7,916,200, a radiological imaging sensor normally comprises a semiconductor imaging chip having a matrix of photosensitive members and linked electronic components, an electronics substrate on which the chip and possibly some other components are mounted, a scintallator covering the chip, and occasionally a fiber-optic plate inserted between the scintillator and the chip. The unit is contained in a resin package from which a connection cable may extend to a system for processing the collected images (except in the case of wireless transmission, in which case a battery is provided, as a rule, in the package). The package conforms as closely as possible to the shape of the chip so as not to create unnecessary bulk. The shape of the chip which is, a priori, rectangular requires the package to have a rectangular shape, which is neither ergonomic nor comfortable for the patient.

Some of the most painful radiographs captured are at the mesial aspect of the premolar bitewing and posterior premolar periapical views. The reason these radiographs are painful to take is that the imaging plate, whether a film or a sensor (which is stiffer and can cause more pain), must be located such that its mesial end is placed as far forward in the patient's mouth as possible to capture the distal aspect of the canine teeth and the mesial aspect of the premolar teeth in a bitewing or periapical view radiograph; and once the patient bites down the edges of the film or sensor dig into the tissue on the anterior ascending aspect of the maxillary palate or the lingual aspect of the anterior mandibular region; thus often causing pain when the mesial aspect of the digital sensor is impinging against these very sensitive anatomic regions during a radiographic exam. When a radiograph is being taken with a sensor with a cord, the sensor must be inserted so that the distal end is located towards the distal aspect of the teeth being imaged and then the mesial end is located at the most mesial aspect of the teeth being imaged. This means that the mesial end of the sensor, which is the end at which the cord from the sensor exits the mouth, is toward the front of the mouth at which the cord exits the mouth. By minimizing dead space at the mesial end MS of a sensor, the procedure for obtaining a radiograph of the patient's posterior teeth is far more comfortable and less painful, and better results are obtained.

US 5 510 623 A discloses a solid state image sensor array having a two block, full-frame, parallel-register structure and a single, shared central read-out register. In an embodiment the image sensor array is a CCD-device that is employed as a part of an X-ray image sensor.

### SUMMARY OF THE INVENTION

The present invention provides a sensor as defined in claim 1, as well as a method as defined in claim 7. Further embodiments of the sensor of the present invention are described in the dependent claims.

The present invention is generally directed to an intraoral radiological imaging sensor having an imaging chip held within a housing. The imaging chip does not have a dead space due to imaging chip control electronics at its mesial side because imaging chip control electronics are either located at its distal side or the imaging chip is substantially free of any imaging chip control electronics located outside of an active pixel array, in which case the imaging chip control electronics are located in a control layer deposited underneath the active pixel array or are contained within imaging chip control electronics for individual pixels in the active pixel array. Such an intraoral radiological imaging sensor is especially useful for capturing a premolar bitewing or posterior periapical view radiograph.

The intraoral radiological imaging sensor has an electronics substrate and can have a flat cable attached to its housing more distant to its mesial side than to its distal side at a cable button connector so that the cable exits the cable button connector toward the mesial side of the generally rectangular shaped sensor.

Accordingly, it is a primary object of the present invention to provide an improved intraoral radiographic sensor that can be used to obtain better radiograph images of some teeth.

This and further objects and advantages will be apparent to those skilled in the art in connection with the drawings and the detailed description of the invention set forth below.

### BRIEF DESCRIPTION OF THE DRAWINGS

Figure 1 is a partial assembly view that illustrates a prior art radiological imaging sensor and its primary components. Figure 1A is a photograph of a prior art radiological imaging sensor showing the orientation between distal and mesial and Figure 1B illustrates a radiograph of the sensor shown in Figure 1A that illustrates the dead space on the mesial end of a typical traditional sensor.
Figure 2 is a side view of a sensor showing a cable button connector located more proximate its distal side than its mesial side.
Figure 3 is a top view cutaway of the sensor of Figure 2 showing certain aspects of a sensor in accordance with a preferred embodiment flat cord exiting the sensor button at a more distal position, with the sensor dead space located at the distal end of the sensor.
Figure 4 illustrates typical loss of imaging area due to dead space for sensor electronics for a typical digital sensor.
Figure 5 illustrates one embodiment of a flat cable useful in a digital sensor.
Figure 6 conceptually illustrates an imaging chip while Figure 6A conceptually illustrates a portion of the active pixel area of Figure 6.

### DETAILED DESCRIPTION OF THE INVENTION

The following glossary is used for the Figures and description which follows herein:

### Glossary:

- 1: radiological imaging sensor
- 2: electronics substrate
- 3: imaging chip
- 4: fiber optic plate
- 5: Csl scintillator
- 6: cable
- 8: dead space attributable to shock absorption material and housing
- 9: dead space attributable to imaging chip control electronics
- 10: cable side housing
- 11: cable button connector
- 15: front side housing
- 21: electronic components
- 32: imaging chip active area
- 33: pixel
- 34: pixel control electronics for individual pixel
- 35: active pixel area
- 38: imaging chip dead space due to chip construction and not due to imaging chip control electronics
- 39: imaging chip dead space due to imaging chip control electronics outside of the active pixel area
- 40: flat cable
- 41: round cable
- 42: connector
- Area A: coverage area A for a standard premolar bitewing (film radiograph)
- Area B: coverage area B shows loss of imaging area due to dead space for imaging chip control electronics for a prior art digital sensor
- CS: cable side
- DS: distal side
- FS: front side
- MS: mesial side

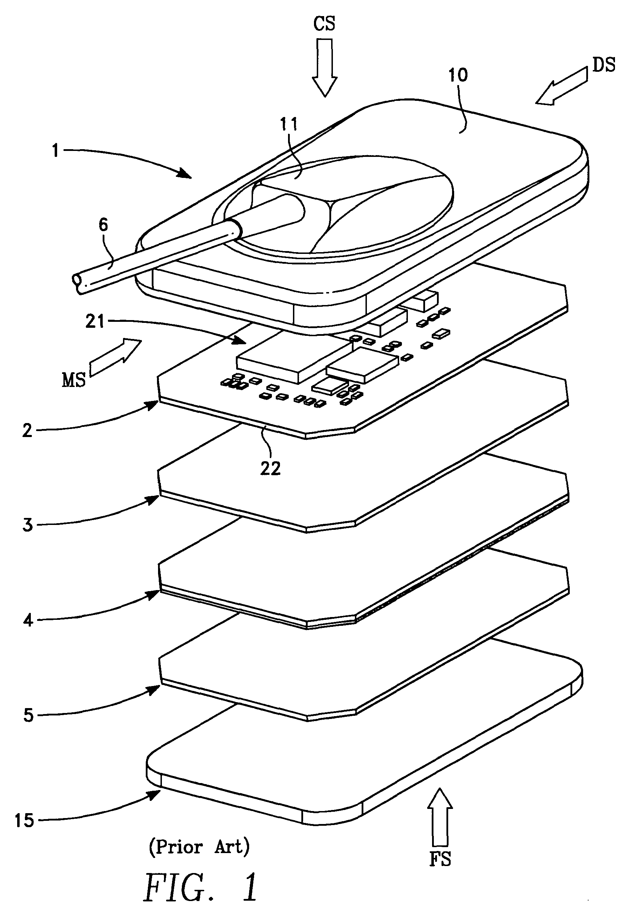

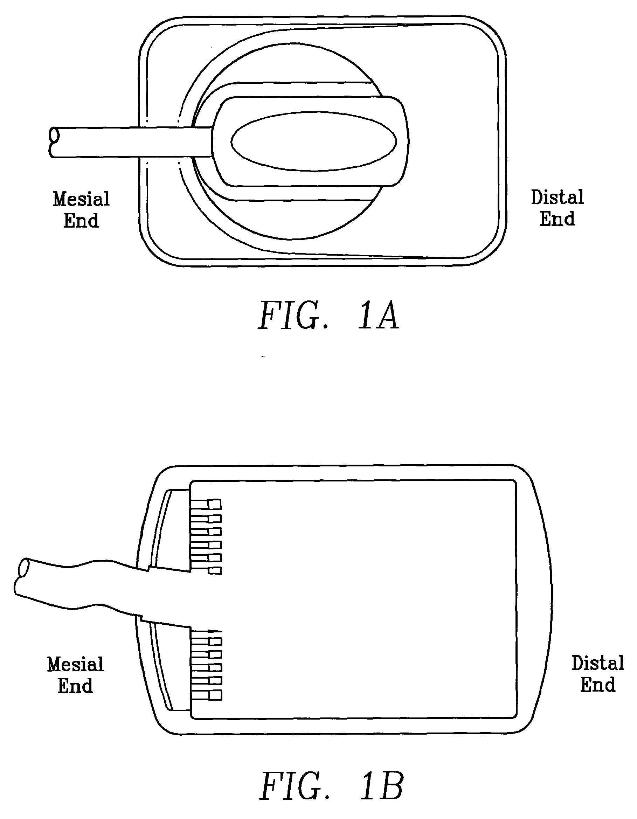

Figure 1 illustrates a prior art radiological imaging sensor 1 that has a cable side housing 10 connected to a front side housing 15 with a cable 6 running out of cable button connector 11 in front side housing 15 toward mesial side MS of sensor 1. Inside the housing, moving from cable side CS down to front side FS, are an electronics substrate, shown generally as 2, electronic components 21 being mounted on the cable side of electronics substrate 2 (e.g., a ceramic or plastic material), an imaging chip, shown generally as 3 (preferably a CMOS imaging chip), a fiber optic plate, shown generally as 4 (which functions as an x-ray filter for improved noise reduction), and a Csl scintillator, shown generally as 5 (optimized for resolution and low noise).

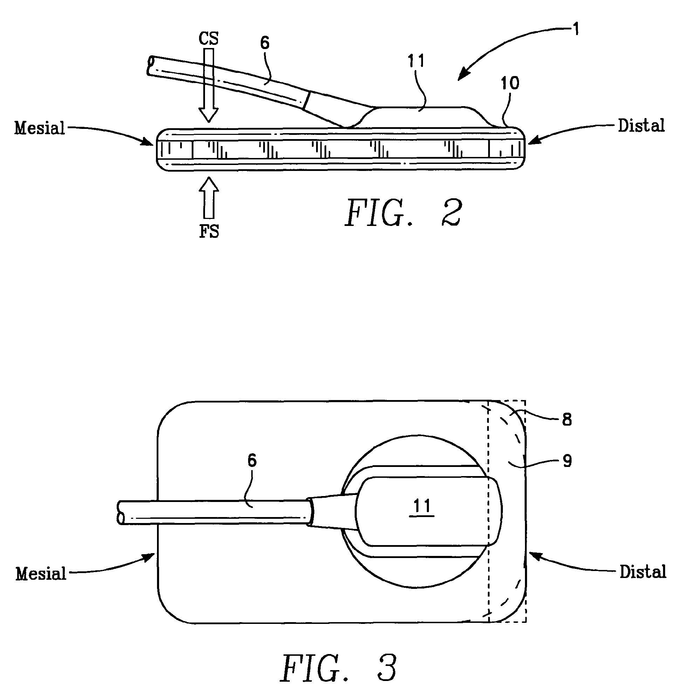

Sensor 1 has a generally rectangular shape, as illustrated in Figure 1. For purposes of the present invention, the shorter sides of the rectangle will always be defined by the direction in which cable 6 exits cable button connector 11. Mesial side MS (see Figure 1) will always be defined as the side toward which cable 6 exits cable button connector 11 while distal side DS will always be defined as the side opposite which cable 6 exits cable button connector 11, even if cable button connector 11 is not centered in cable side housing 10 (see Figure 2).

In accordance with the present invention, it is especially preferred that cable button connector 11 be located more proximate to the distal side DS than centered (see Figure 2). The reason such placement is preferred is that it allows more room for cable 6 coming out of cable button connector 11 to twist or turn when the sensor is used in some locations of a patient's mouth, thus reducing stress on the connection between cable 6 and cable button connector 11, which must be watertight.

In typical prior art sensors, electronics substrate 2 has a shock absorption material (not shown) around the periphery of ceramic material 22. The space occupied by shock absorption material, as well as space occupied the sensor housing (once cable and front side housings 10 and 15 are assembled together), creates a dead space 8 (see Figure 3) in which a radiographic image is not obtained, and the size of this dead space will typically be no less than 2 mm. In addition to dead space 8, a second dead space 9 is created by imaging chip control electronics located on mesial side MS of imaging chip 3 in presently available sensors, which can represent another 4 mm or more of additional dead space.

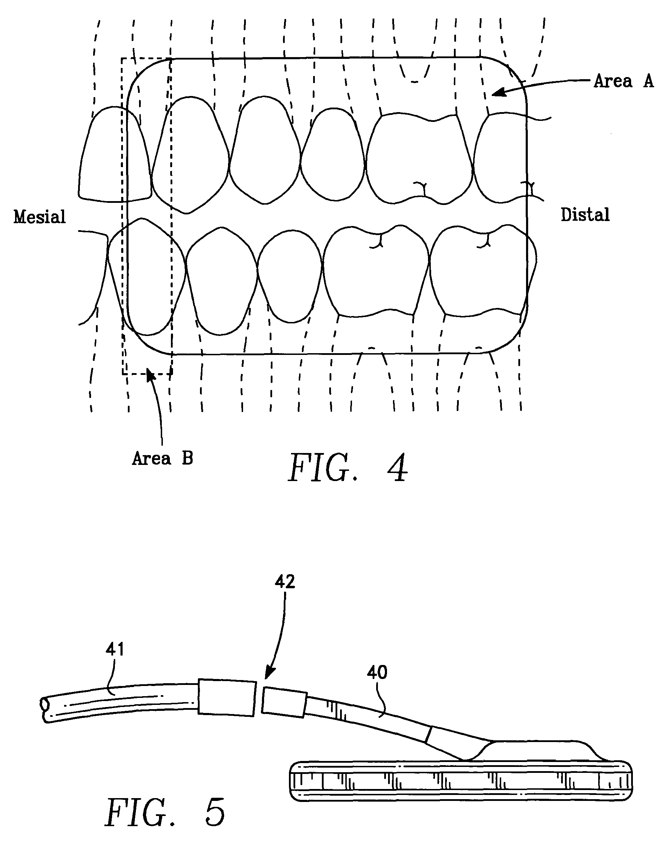

The combined effect of dead spaces 8 and 9 in currently available intraoral radiographic sensors is an inability to duplicate the same coverage area in a patient's oral anatomy as x-ray film when placed in the exact same position relative to the patient's teeth. This problem is due to the intrinsic design and layout of all digital intraoral sensors, with regard to the placement of the dead space, which is created as a by-product by parts of the electronics on the sensor. Significantly, this 4-8mm dead space is approximately the width of half to a whole canine, or premolar tooth, as shown in Figure 4.

Figure 4 illustrates a coverage Area A for a standard premolar bitewing film radiograph. Within Area A, at its mesial side, is another Area B. Area B illustrates a typical loss of imaging area due to dead space for sensor electronics for a digital sensor. Accordingly, Figure 4 illustrates that if a digital dental sensor is placed in the exact position as the x-ray film, the resultant image will not show the first 4-8mm of the mesial end of the patient's anatomy.

The first prior art solid-state sensors had the cord exiting off the mesial edge. It was along this edge of the associated imaging chip that the control electronics were placed, in close proximity to the cord for efficiency of wiring to the cord. As the cord attachment moved to the back side of the sensor allowing for easier bending of the cable and patient comfort when the sensor was used in a vertical orientation in the patient's mouth, the placement of electronics and resulting imaging dead-space, remained the same. This became part of the industry standard in design and was never questioned because design engineers only thought about ease of fabrication, ease of connection, and minimizing signal loss in the placement of the electronics. No thought was given to clinical ergonomic issues.

However, the present invention addresses clinical ergonomic issues, which represents a major change in intraoral sensor design, by moving dead space 9 to distal side DS of imaging chip 3 so that any dead space located on the mesial side of the sensor is kept to a bare minimum attributable solely to shock absorption material and the housing. Such a sensor design is shown in Figure 3 which illustrates a cable side view of an electronics substrate 2 with dead space 9 being located at distal side DS, not mesial side MS. For purposes of orientation, cable 6 and cable button connector 11 are shown as trace lines in Figure 3.

Locating dead space 9 at distal side DS, contrary to current practices and traditional wisdom, allows a dental practitioner to capture images not currently obtainable because Area B of Figure 4, which represents a loss of imaging area due to dead space, is minimized, thus allowing greater capture of teeth located in the mesial area of a radiograph capture, which is especially important when a bitewing or periapical radiograph is being taken of the canine and premolar teeth.

In this regard, some of the most painful radiographs captured are the premolar bitewing and posterior periapical views. The reason these radiographs are painful to take is that the imaging plate, whether a film or a sensor (which is stiffer and can cause more pain), must be located such that its mesial end is placed as far forward in the patient's mouth as possible to capture the distal aspect of the canine teeth and the mesial aspect of the premolar teeth in a bitewing or periapical view radiograph; and once the patient bites down the edges of the film or sensor dig into the tissue on the anterior ascending aspect of the maxillary palate or the lingual aspect of the anterior mandibular region; thus often causing pain when the mesial aspect of the digital sensor is impinging against these very sensitive anatomic regions during a radiographic exam. When a radiograph is being taken with a sensor with a cord, the sensor must be inserted to that the distal end is located towards the distal aspect of the teeth being imaged and then the mesial end is located at most mesial aspect of the teeth being imaged. By minimizing dead space at the mesial end MS of a sensor, the procedure for obtaining a radiograph of the patient's posterior teeth is far more comfortable and less painful, and better results are obtained.

Accordingly, a sensor in which dead space in its mesial end is minimized, represents a significant advance over the prior art and allows dental practitioners to obtain much better radiographs of all teeth being radiographed.

It should be noted that the embodiment disclosed are not limited solely to locating dead space 9 at distal side DS and, in alternative embodiments, it is contemplated that, as much as is possible, the imaging chip control electronics for digital sensors should be moved so that they lie within the actual imaging area of imaging chip 3, whether done by sacrifice of active imaging area within individual pixels or by depositing the imaging chip control electronics as a layer upon which the active imaging area is then deposited.

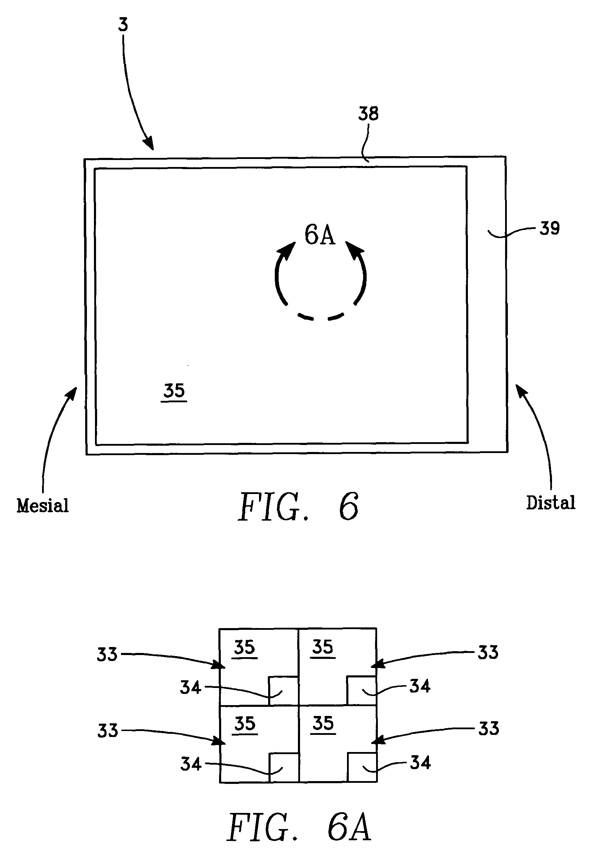

Figure 6 illustrates an imaging chip 3 which has an imaging chip active area 32 made up of individual pixels 33. Each individual pixel 33 has its own pixel control electronics 34 and active pixel area 35. Imaging chip 3 also has an imaging chip dead space 38 due to chip construction and not due to imaging chip control electronics. In current imaging chips, there is also a dead space 39 (although it is located at mesial side MS, not distal side DS) due to imaging chip control electronics outside of the active pixel area. The imaging chip control electronics can perform many functions, including intra and inter pixel imaging chip control. Thus, in accordance with the alternative embodiments just noted, dead space 39 of Figure 6 can be eliminated, and capture efficiency can be improved, by moving all imaging chip control electronics to a layer that lies underneath the active layer of the imaging chip relative to front side FS. This can be done by first depositing an imaging chip control electronics layer and then depositing the active imaging area on top of the already deposited electronics layer. Alternatively, or in combination with such a deposited imaging chip control layer, capture efficiency can be increased by including imaging chip control electronics in each pixel's pixel control electronics 34, even if some active pixel area 35 must be sacrificed.

Another aspect focuses on minimizing discomfort associated with obtaining radiographs of teeth with radiological imaging sensors that include a connection cable by changing the shape of the connection cable from round or circular to an asymmetric shape that is substantially wider than its height, preferably at least two or more times wider than its height, examples of which might be ovoid or flat. Such an improved cable, for the remainder of this description, will be referred to as a flat cable.

A flat cable will be easier to fit to a patient's mouth while certain radiographs are taken because it reduces cord bulk and bite interference. Rather than having to bite down with a circular cord running out between the patient's teeth, the patient will now have to bite down on a flat cord that creates less of a gap between teeth, thus increasing comfort and imaging coverage of the sensor. Also, use of a flat cord may reduce the thickness of cable button connector 11, which should also increase patient comfort and easier placement of the sensor in the patient's mouth.

Accordingly, a sensor in which a flat cord is implemented represents a significant advance over the prior art and allows dental practitioners to obtain much better radiographs of all teeth being radiographed and increased patient comfort.

Current connection cables are round and designed to meet applicable standards for USB connections as well as UL and other applicable standards. The desire to meet USB standards stems, at least in part, for ease of use and the ability to quickly and easily connect with computers.

A flat cable suitable for use can meet USB standards, but it need not necessarily do so. The key design criteria is to reduce the thickness of the flat cable that must fit between upper and lower teeth when certain radiographs are being taken. One possible alternative of a flat cable uses a short flat cable 40, with a length of approximately one meter or less (not shown to scale), that can be connected to a round USB compliant cable 41 by a small connector, an example of which is shown generally in Figure 5 as 42. (Note that connector 42 can be comprised of a female end on one cable and a male end on the other cable).

When a flat cord is combined with reduced dead space in the mesial end of a sensor, the result is a much improved sensor which provides increased comfort in a patient's mouth, improved image coverage at the mesial end, reduced stress on the cord attachment to the sensor housing, improved cord durability and reduce cord bulk and interference.

In the following, further embodiments will be described:

According to a first further embodiment, an intraoral radiological imaging sensor may comprise an electronics substrate and an imaging chip held within a housing, said imaging chip having electronics that create a dead space; and a cable attached to the housing at a cable button connector; wherein the sensor has a generally rectangular shape with a mesial side toward which the cable exits the cable button connector and a distal side opposite which the cable exits the cable button connector; and wherein a majority of the dead space is created in the distal side of the sensor.

In an exemplary aspect of the first further embodiment, the mesial side of the sensor does not have a second dead space created by electronics of the imaging chip.

In another exemplary aspect of the first further embodiment, substantially all of the dead space is created in the distal side of the sensor.

In yet another exemplary aspect of the first further embodiment, the sensor has a mesial side dead space of approximately 2 mm or less.

In still another exemplary aspect of the first further embodiment, the cable is a flat cable. E.g., the cable button connector may be mounted to a cable side of the housing more distant to the mesial side than to the distal side.

According to a second further embodiment, an intraoral radiological imaging sensor is provided, comprising an imaging chip held within a housing, said imaging chip being substantially free of a dead space due to any imaging chip control electronics outside of an active pixel array.

In a first aspect of the second further embodiment, a mesial side of the imaging chip is free of any dead space due to said any imaging chip control electronics outside of the active pixel array.

In a first example of the first aspect, the active pixel array may be further comprised of a plurality of pixel control electronics for use in individual pixels in the active pixel array.

Optionally, in this first example, the imaging chip does not contain any imaging chip control electronics for use in controlling the imaging chip that are not contained within the active pixel array.

According to a first alternative, the plurality of pixel control is deposited in a control layer of electronics formed underneath the active pixel array.

According to a second alternative, the imaging chip control electronics is contained within the plurality of pixel control electronics.

According to a third further embodiment, there is provided a method for capturing a premolar bitewing or posterior periapical view radiograph through use of an intraoral radiological imaging sensor having an imaging chip held within a housing, the method comprising the steps of:
locating the intraoral radiological imaging sensor such that its mesial end is placed as far forward in the patient's mouth as possible to capture the distal aspect of the canine teeth and the mesial aspect of the premolar teeth in a bitewing or periapical view radiograph; and
obtaining the radiograph;
wherein there is substantially no dead space at the mesial end of the intraoral radiological imaging sensor due to imaging chip control electronics for an active pixel array of the imaging chip.

While the invention has been described herein with reference to certain preferred embodiments, those embodiments have been presented by way of example only, and not to limit the scope of the invention. Additional embodiments thereof will be obvious to those skilled in the art having the benefit of this detailed description.

## Claims

1. An intraoral radiological imaging sensor (1), comprising:
an electronics substrate (2) and an imaging chip (3) held within a housing (10), said imaging chip having electronics that create a dead space (9); and
a cable (6) attached to the housing (10) at a cable button connector (11);
wherein the sensor (1) has a generally rectangular shape with a mesial side toward which the cable (6) exits the cable button connector (11) and a distal side opposite which the cable exits the cable button connector; and
**characterised in that** a majority of the dead space (9) is created in the distal side of the sensor (1).

2. The intraoral radiological imaging sensor (1) of claim 1, wherein the mesial side of the sensor (1) does not have a second dead space created by electronics of the imaging chip (3).

3. The intraoral radiological imaging sensor (1) of claim 1, wherein substantially all of the dead space (9) is created in the distal side of the sensor (1).

4. The intraoral radiological imaging sensor (1) of claim 1, wherein the sensor (1) has a mesial side dead space of approximately 2 mm or less.

5. The intraoral radiological imaging sensor (1) of claim 1, wherein the cable (6) is a flat cable.

6. The intraoral radiological imaging sensor (1) of claim 5, wherein the cable button connector (11) is mounted to a cable side of the housing (10) more distant to the mesial side than to the distal side.

## Patentansprüche

1. Ein intraoraler radiologischer Bildsensor (1), aufweisend:
ein Elektronik-Substrat (2) und einen in einem Gehäuse (10) gehaltenen Bildchip (3), wobei der Bildchip Elektronik hat, die einen toten Raum (9) bildet; und
ein Kabel (6), das an einem Kabelknopfverbinder (11) an dem Gehäuse (10) angebracht ist;
wobei der Sensor (1) eine im Allgemeinen rechteckige Form hat, mit einer mesialen Seite, zu der hin das Kabel (6) aus dem Kabelknopfverbinder (11) austritt, und einer distalen Seite, gegenüber von welcher das Kabel aus dem Kabelknopfverbinder austritt; und
**gekennzeichnet dadurch, dass** eine Mehrheit des toten Raums (9) in der distalen Seite des Sensors (1) gebildet ist.

2. Der intraorale radiologische Bildsensor (1) nach Anspruch 1, wobei die mesiale Seite des Sensors (1) keinen zweiten toten Raum hat, der von Elektronik des Bildchips (3) gebildet ist.

3. Der intraorale radiologische Bildsensor (1) nach Anspruch 1, wobei im Wesentlichen der gesamte tote Raum (9) in der distalen Seite des Sensors (1) gebildet ist.

4. Der intraorale radiologische Bildsensor (1) nach Anspruch 1, wobei der Sensor einen mesiale-Seite-toter-Raum von etwa 2 mm oder weniger aufweist.

5. Der intraorale radiologische Bildsensor (1) nach Anspruch 1, wobei das Kabel (6) ein Flachkabel ist.

6. Der intraorale radiologische Bildsensor (1) nach Anspruch 5, wobei der Kabelknopfverbinder (11) an einer Kabelseite des Gehäuses (10) montiert ist, weiter entfernt von der mesialen Seite als von der distalen Seite.

## Revendications

1. Capteur d'imagerie (1) radiologique intra-oral, comprenant :
un substrat (2) pour électronique et une puce d'imagerie (3) contenue dans un boîtier (10), ladite puce d'imagerie présentant une électronique qui crée un espace mort (9) ; et
un câble (6) rattaché au boîtier (10) au niveau d'un connecteur (11) pour fiche de câble ;
dans lequel le capteur (1) présente une forme généralement rectangulaire avec un côté mésial en direction duquel le câble (6) sort du connecteur (11) pour fiche de câble et un côté distal à l'opposé duquel le câble sort du connecteur pour fiche de câble ; et
**caractérisé en ce que** la plus grande partie de l'espace mort (9) est créée dans le côté distal du capteur (1).

2. Capteur d'imagerie (1) radiologique intra-oral selon la revendication 1, dans lequel le côté mésial du capteur (1) ne présente pas de deuxième espace mort créé par l'électronique de la puce d'imagerie (3).

3. Capteur d'imagerie (1) radiologique intra-oral selon la revendication 1, dans lequel substantiellement tout l'espace mort (9) est créé dans le côté distal du capteur (1).

4. Capteur d'imagerie (1) radiologique intra-oral selon la revendication 1, dans lequel le capteur (1) présente un espace mort côté mésial d'environ 2 mm ou moins.

5. Capteur d'imagerie (1) radiologique intra-oral selon la revendication 1, dans lequel le câble (6) est un câble plat.

6. Capteur d'imagerie (1) radiologique intra-oral selon la revendication 5, dans lequel le connecteur (11) pour fiche de câble est monté à un côté câble du boîtier (10) plus éloigné du côté mésial que du côté distal.
